Europäisches Patentamt

European Patent Office

Office européen des brevets

(19)

(11) Numéro de publication : **0 047 557**
**B1**

(12) **FASCICULE DE BREVET EUROPÉEN**

(45) Date de publication du fascicule du brevet :
04.03.87

(51) Int. Cl.⁴ : **A 61 F   2/00, A 61 L 27/00**

(21) Numéro de dépôt : **81200971.0**

(22) Date de dépôt : **01.09.81**

(54) **Perfectionnement à la composition d'un matériel d'implantation.**

(30) Priorité : **10.09.80 BE 6047262**

(43) Date de publication de la demande :
**17.03.82 Bulletin 82/11**

(45) Mention de la délivrance du brevet :
**04.03.87 Bulletin 87/10**

(84) Etats contractants désignés :
**AT CH DE FR GB IT LI LU NL SE**

(56) Documents cités :
**EP-A- 0 029 787**
**GB-A- 1 588 002**
**ENGINEERING IN MEDICINE, vol. 9, no. 1, janvier 1980, LONDRES (GB) B. GREGORY et al.: "The corrosion fatigue of a molybdenum ion-plated ortho-paedic En58J stainless steel", pages 3-7**
**ENGINEERING IN MEDICINE, vol. 7, no. 3, juillet 1978, LONDRES (GB) J. BRETTLE et al.: "The corrosion properties of currently used and potentially useful surgical implant materials", pages 142-150**
**MEDICAL PROCESS TECHNOLOGY, vol. 5, no. 4, mai 1978, Springer-Verlag 1978 J. OHNSORGE et al.: "Surface Investigations of Oxide Layers on Cobalt-Chromium-Alloyed Orthopedic Implants Using ESCA Technique", pages 171-177**

(73) Titulaire : **Lempereur, Léon**
**15 rue Tumelaire**
**B-6000 Charleroi (BE)**

(72) Inventeur : **Lempereur, Léon**
**15 rue Tumelaire**
**B-6000 Charleroi (BE)**

(74) Mandataire : **Dellicour, Paul**
**Office de Brevets E. Dellicour rue Fabry 18/012**
**B-4000 Liège (BE)**

EP 0 047 557 B1

## Description

La présente invention concerne le matériel d'implantation dans le corps humain ainsi que dans les corps d'animaux et les milieux biologiques, pouvant servir à diverses utilisations, notamment à l'ostéosynthèse, et est relative à sa composition et à son traitement.

Dans les cultures de tissus isolés, l'acier inoxydable est très peu cytotoxique mais chez l'animal et chez l'homme, cet alliage présente le danger de la rupture fragile par corrosion sous tension ; cet alliage ainsi que les autres alliages et matériaux présentent en plus divers inconvénients, quand ils sont implantés chez l'homme ou l'animal ou les milieux biologiques. Par contre, l'acier au carbone traité présente des propriétés inverses : chez l'animal et chez l'homme, par un effet de surface, il se recouvre d'un enduit noirâtre annulant la cytotoxicité.

On part donc, suivant l'invention, d'un matériel d'implantation servant notamment à l'ostéosynthèse, réalisé en un acier au carbone ayant subi comme traitement un polissage mécanique car le polissage électrolytique semble retarder l'apparition du dépôt protecteur et surtout est lié à l'apparition de corrosion en profondeur.

Le matériel d'implantation suivant l'invention est caractérisé en ce qu'il est constitué d'un acier au carbone exempt d'impuretés de surface et très pauvre en corps étrangers au fer et au carbone, et en ce que le traitement consistant en un polissage mécanique progressif est exécuté avec des grains d'abrasif au maximum jusqu'au n° 600 tout en atteignant toujours le n° 400.

L'acier au carbone utilisé pour la fabrication d'endoprothèses est suivant l'invention nettoyé à fond pour éliminer les impuretés de surface. Il ne doit comporter que peu d'impuretés de structure et doit donc être très pauvre en corps étrangers au fer et au carbone, ces deux produits étant considérés comme des produits biologiques. Le fer et le carbone étant contenus dans le corps humain, on peut considérer qu'un matériel d'implant suivant l'invention est lui-même un produit biologique.

L'avantage du matériel en acier au carbone caractérisé suivant l'invention consiste en ce qu'il est capable, aux moindres frais, de répondre parfaitement aux forces statiques et dynamiques du corps humain, d'une manière durable en participant facilement sans causer aucun trouble à la vie normale de l'hôte dans son ensemble et des tissus, quels qu'ils soient, où il est implanté.

Le traitement consistant en un polissage mécanique caractérisé suivant l'invention réalise un aplatissement des grains, une diminution des joints de grains permettant encore un accrochage ; il s'agit ici d'un problème de rugosités.

Dans un matériel d'implantation suivant l'invention un enduit noirâtre s'accroche facilement endéans les trois semaines, empêchant les actions nocives de l'hôte sur l'implant d'une part et de l'implant sur l'hôte d'autre part.

L'obtention de cette couche noirâtre empêche aussi l'apparition de la réaction de rejet.

On obtient ainsi un bon recouvrement sur la plaque métallographique.

On prévoit également suivant l'invention, pour la facilité, de réaliser une oxydation préalable, de manière à obtenir une couche noirâtre très mince pour pouvoir adhérer, l'épaisseur étant de l'ordre de la longueur d'onde du rayonnement bleu.

D'autre part, dès que le traitement de polissage mécanique et l'oxydation préalable sont terminés, il convient de maintenir l'implant à l'abri de l'air et de l'humidité jusqu'à son utilisation.

Un matériel d'implantation suivant l'invention présente l'avantage d'être plus solide et mieux accepté physiologiquement par le blessé que les implants actuels qui ont comme inconvénients très sérieux : rejet, inflammation et casse, d'où immobilisation beaucoup plus longue.

## Revendications

1. Matériel d'implantation dans le corps humain ainsi que dans les corps d'animaux et les milieux biologiques, caractérisé en ce qu'il est constitué d'un acier au carbone exempt d'impuretés de surface et très pauvre en corps étrangers au fer et au carbone, et en ce que cet acier au carbone a subi comme traitement un polissage mécanique progressif exécuté avec des grains d'abrasif au maximum jusqu'au n° 600.

2. Matériel d'implantation suivant la revendication 1, caractérisé par une oxydation préalable, l'épaisseur de la couche noirâtre obtenue étant de l'ordre de la longueur d'onde du rayonnement bleu.

## Claims

1. Implant material for the human body as well as for animal bodies and biologic media, characterised in that it consists of carbon steel free of surface impurities and contains very few foreign matters other than iron and carbon, and that said carbon steel has been treated by means of gradual mechanical polishing using abrasive material with a grain not exceeding n° 600.

2. Implant material according to claim 1, characterised by preliminary oxidation, the thickness of the darkisch layer obtained corresponding to about the wavelength of blue in the radiation spectrum.

## Patentansprüche

1. Material zur Implantation in den menschlichen Körper, sowie in Tierkörper und biologische Medien, dadurch gekennzeichnet, daß es aus Kohlenstoffstahl besteht, der frei von Oberflächenverunreinigungen und sehr arm an eisen- und kohlenstoffhaltigen Fremdkörpern ist, und daß dieser Kohlenstoffstahl einer progressiven mechanischen Polierbehandlung unterworfen wurde, die mit Schleifkörnern bis maximal Nr. 600 ausgeführt wurde.

2. Material zur Implantation gemäß Anspruch 1, gekennzeichnet durch eine vorherige Oxydation, wobei die Dicke der erhaltenen schwärzlichen Schicht ungefähr gleich der Wellenlänge von blauem Licht ist.